# EUROPEAN PATENT APPLICATION

(11) **EP 4 201 407 A1**
(43) Date of publication of application: **28.06.2023**
(21) Application number: 21858593.3
(22) Date of filing: 18.08.2021
(51) Int. Cl.: A61K 31/541, A61P 31/04

(54) **MITOCHONDRIA-TARGETED ANTIOXIDANT AS AGENT FOR TREATING PATHOLOGIC INFLAMMATION CAUSED BY MABC-R INFECTION**

(30) Priority: 19.08.2020 KR 20200103849
(71) Applicant: MitoImmune Therapeutics Inc., Seoul 06123 (KR)
(72) Inventor: KIM, Soon Ha, Yongin-si Gyeonggi-do 16803 (KR); JO, Eun Kyeong, Daejeon 34963 (KR); KIM, Young Jae, Daejeon 35207 (KR)
(74) Representative: Plasseraud IP
(86) International application number: PCT/KR2021/010982
(87) International publication number: WO 2022/039506

(57) **Abstract**

The present invention relates to a pharmaceutical composition for use in the prevention or treatment of nontuberculous mycobacterium infectious diseases, containing, as an active ingredient, a compound represented by Chemical Formula 1 or a pharmaceutically acceptable salt thereof. The compound represented by Chemical Formula 1 or a pharmaceutically acceptable salt thereof, according to the present invention, can be used in the prevention or treatment of nontuberculous mycobacterium (NTM) infectious diseases.

## Description

### [Technical Field]

The present invention relates to a pharmaceutical composition for preventing or treating nontuberculous mycobacterium (NTM) infectious diseases, containing, as an active ingredient, a compound represented by the following Chemical Formula 1 or a pharmaceutically acceptable salt thereof.

The present invention claims priority based on the filing date Aug. 19, 2020, application number 10-2020-0103849, title "Mitochondria-targeted antioxidant as agent for pathological inflammation caused by Mabc-R infection", the entire contents of which are incorporated herein as part of the present invention.

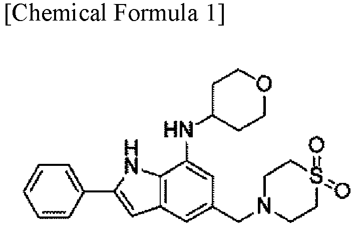

### [Background Art]

*Mycobacterium abscessus* (Mabc) is a nontuberculous mycobacterium that causes numerous human infections, usually associated with lung diseases (References [1, 2]). *Mycobacterium abscessus* is a subspecies of the Mabc complex that includes *Mycobacterium abscessus, Mycobacterium massiliense* and *Mycobacterium bolletii,* and has major clinical implications due to antibiotic resistance (References [2, 3]). Mabc often causes pulmonary infections in patients with immune deficiencies, such as cystic fibrosis, and may threaten immunocompetent individuals (References [4, 5]). Mabc is divided into two morphological types, smooth and rough variants, depending on the colony phenotype (References [6, 7]). The Mabc-rough (Mabc-R) variant is more virulent and invasive compared to Mabc smooth forms. Mabc-R has very minimal glycopeptidolipids (GPLs), which mask the underlying phosphatidyl-myo-inositol mannosides and induce innate immune and inflammatory responses (Reference [8]).

Although several risk factors (for example, old age, immune-modulating agents, underlying lung diseases) related to NTM diseases have been reported (References [5, 9]), the host factors that are associated with pathogenesis during Mabc infection are largely unknown.

Mitochondria are essential intracellular organelles for oxidative energy metabolism, adenosine triphosphate (ATP) production, and calcium homeostasis (Reference [10]). In mitochondria, numerous proteins/enzymes associated with respiratory chain reaction, dynamics, and energy metabolism are regulated by lysine acetylation (Reference [10]).

Meanwhile, sirtuin 3 (SIRT3) is a major deacetylase in mitochondria (Reference [11]) and is known to affect mitochondrial functions through nicotinamide adenine dinucleotide (NAD+)-dependent protein deacetylation (Reference [12]). The function of SIRT3 in metabolic tissues has been studied and accumulated data implies that SIRT3 plays a crucial role in non-metabolic cells and tissues, including the lungs and immune cells (References [10 and 12 to 15]).

The present inventors confirmed through recent studies that SIRT3 is required for the host's innate defense against *Mycobacterium tuberculosis* (Mtb) infection. Importantly, SIRT3 contributes to mitochondrial homeostasis and xenophagy activation in macrophages during Mtb infection (Reference [16]). However, observations were inconsistent regarding the function of SIRT3 in host defenses against various bacterial and fungal infections, including *Escherichia coli* and *Klebsiella pneumoniae* infections (Reference [15]). In addition, recent studies showed that SIRT3/5 double knockout (KO) mice showed improved resistance to *Listeria* infection (Reference [17]). Accordingly, to date, the putative role of SIRT3 in NTM infection is unknown. Furthermore, recent advances and emerging data have revealed SIRT3 modulators, which may ultimately be used as promising drugs for control of numerous human disorders (Reference [12]). In this regard, the therapeutic potential of a SIRT3-targeted therapeutic agent could be validated as a potential drug target for the treatment of Mabc infection *in vivo.*

### [Disclosure]

### [Technical Problem]

An object of the present invention is to provide a pharmaceutical composition for use in the prevention or treatment of nontuberculous mycobacterium infectious diseases, containing, as an active ingredient, a compound represented by Chemical Formula 1 or a pharmaceutically acceptable salt thereof.

### [Technical Solution]

Under the above-described background, the present inventors confirmed whether mitochondrial homeostasis is important for host protection against Mabc infection and whether SIRT3-mediated, mitochondria-targeted therapy is beneficial for controlling Mabc infection *in vivo.* As a result, the present inventors confirmed that Mabc infection led to a decrease in expression of SIRT3, mitochondrial reactive oxygen species (ROS) generation, and mitochondrial dysfunction in macrophages, and SIRT3 deficiency increased bacterial loads, mitochondrial ROS and dysfunction, and pathological inflammation in response to Mabc infection.

Furthermore, it was confirmed that the compound represented by Chemical Formula 1, which is a mitochondrial ROS scavenger, has a therapeutic effect against Mabc infection through amelioration of pathological inflammation and suppression of mitochondrial ROS. More specifically, in an exemplary embodiment, it was confirmed that the compound represented by Chemical Formula 1 has an effect of improving the SIRT3 mRNA level, which has decreased due to nontuberculous mycobacterium infection.

Therefore, the present invention provides a pharmaceutical composition for use in the prevention or treatment of nontuberculous mycobacterium infectious diseases, containing, as an active ingredient, a compound represented by Chemical Formula 1 or a pharmaceutically acceptable salt thereof.

In the present specification, the compound represented by Chemical Formula 1, 5-[(1,1-dioxido-4-thiomorpholinyl)methyl]-2-phenyl-*N*-(tetrahydro-2*H*-pyran-4-yl)-1*H*-indol-7-amine is a compound disclosed through International Patent Publication No. WO2009-025477,, and is a substance known to exhibit preventive, therapeutic and ameliorative effects on necrosis and related diseases.

According to WO2009-025477, the compound of Chemical Formula 1 is known to exhibit preventive or therapeutic and ameliorative effects on necrosis and related diseases. According to WO2009-025477, the necrosis and related diseases include acute/chronic liver diseases (for example, hepatitis, hepatic fibrosis, and liver cirrhosis), neurodegenerative diseases (for example, dementia, Parkinson's disease, and Huntington's disease), ischemic heart disease, reperfusion injury (Korean Patent No. 10-1325272), ischemic stroke or ischemic injury, pancreatitis, bacterial/viral sepsis, diabetes mellitus or diabetes complications, diabetic vascular disease [in particular, these types of diabetes are caused by pancreatic cell-destroying substances, and mediated by viruses, hyperglycemia, fatty acids, diet, toxins, streptozotocin, and the like], necrotizing proctitis, cystic fibrosis, rheumatoid arthritis, degenerative arthritis, nephropathy, bacterial infection, viral infection (for example, HIV), multiple sclerosis, leukemia, lymphoma, neonatal respiratory distress syndrome, asphyxia, tuberculosis, endometriosis, angiasthenia, psoriasis, frostbite, steroid side effects, gangrene, tenderness, hemoglobinuria, burns, hyperthermia, Crohn's disease, celiac disease, compartment syndrome, spinal cord injury, glomerulonephritis, muscular dystrophy, inherited metabolic disease, mycoplasma disease, anthrax, Andersen's disease, congenital mitochondrial disease, phenylketonuria, placental infarction, syphilis, aseptic necrosis, and the like. In addition, necrosis and associated diseases caused by drugs and toxic substances are selected from the group consisting of necrosis associated with alcoholism, the exposure to, and/or administration and/or self-administration of, cocaine, drugs (for example, paracetamol), antibiotics, anti-cancer agents, Adriamycin, puromycin, bleomycin, NSAID, cyclosporine, toxic chemicals (for example, carbon tetrachloride, cyanide, methanol, and ethylene glycol), poison gas, agrochemicals, heavy metals (for example, lead, mercury, and cadmium), or injury due to the exposure to radioactivity/UV and associated necrosis thereof.

Furthermore, the compound of Chemical Formula 1 is expected to additionally exhibit preventive or therapeutic and ameliorative effects on acute/chronic kidney disease, traumatic brain injury, amyotrophic lateral sclerosis which is a neurodegenerative disease, necrotizing colitis, viral infection (for example, SARS-CoV), skin diseases including psoriasis and allergic dermatitis, organ preservation/organ transplantation (see Korean Patent Nos. 10-10983 and 10-1941004), and the like among necrosis and related diseases.

Further, a pharmaceutical composition including the compound of Chemical Formula 1 has a function of regulating intracellular calcium, and may ameliorate ER stress and mitochondrial dysfunction caused by abnormal intracellular calcium levels. Therefore, the pharmaceutical composition including the compound of Chemical Formula 1 is expected to exhibit preventive or therapeutic and ameliorative effects on related diseases. Related diseases are as follows.

Inflammatory pulmonary diseases including acute lung injury syndrome/acute pulmonary disease, pneumonia, tuberculosis, asthma, pulmonary arterial hypertension, chronic obstructive pulmonary disease, idiopathic pulmonary fibrosis and cystic fibrosis (see mitochondrial dysfunction in fibrotic diseases. cell Death Discov. 2020 Sep 5;6:80*.* Mitochondrial dysfunction in lung aging and diseases. Eur Respir Rev. 2020 Oct 15;29(157):200165*.,* and see Korean Patent No. 10-1636563)

Demyelinating diseases including demyelination and amyotrophic lateral sclerosis (ALS), hypertension including pulmonary artery hypertension, stroke, prion disease, epilepsy, ataxia, migraines, cognitive decline, seizures, tremors, mental illness (for example, depression) (see Neuronal and glial calcium signaling in Alzheimer's disease. Cell Calcium. Oct-Nov 2003;34(4-5):385-97*.* Mitochondrial disorders: challenges in diagnosis & treatment. Indian J Med Res. 2015 Jan;141(1):13-26*.*)

Insulin resistance, hyperlipidemia, atherosclerosis, inflammatory bowel disease (IBD) including Crohn's disease and ulcerative colitis, Various cancers and cancer metastases (see reticulum stress and oxidative stress in cell fate decision and human disease. AntioxidRedox Signal. 2014 Jul 20;21(3):396-413*.)*

Visual impairment-related diseases (for example, retinitis pigmentosa, optic neuropathy, cataract, and glaucoma), anemia, cholestasis, hypoparathyroidism, pancytopenia, pancreatic disorder, lactic acidosis, lactic acidemia, hearing loss, short stature, ileus, cardiac conduction defects, cardiomyopathy, endometriosis, infertility, and premature menopause (see Mitochondrial diseases: the contribution of organelle stress responses to pathology. Nat Rev Mol Cell Biol. 2018 Feb;19(2):77-92*.* Seminars in medicine of the Beth Israel Hospital, Boston. Mitochondrial DNA and disease. N Engl J Med. 1995 Sep 7; 333(10): 638-44*.* Mitochondrial injury and dysfunction in hypertension-induced cardiac damage. Eur Heart J. 2014 Dec 7; 35(46): 3258-3266*.)*

Muscular dystrophy including limb girdle/Becker muscular dystrophy (LGMD/BMD) and Duchenne muscular dystrophy (DMD) (see Duchenne muscular dystrophy is associated with the inhibition of calcium uniport in mitochondria and an increased sensitivity of the organelles to the calcium-induced permeability transition.

### Biochim Biophys Acta Mol Basis Dis. 2020 May 1;1866(5):165674.)

Aging and aging-related diseases (see Interrelation between ROS and Ca²⁺ in aging and age-related diseases. Redox Biology. 2020; 6:101678*.)*

According to WO2009-025477, the pharmaceutical composition including the compound of Chemical Formula 1 not only exhibits liver protection and liver function improving effects, but also a preventive or therapeutic effect on acute and chronic liver diseases such as chronic hepatic diseases such as fatty liver, hepatic fibrosis and hepatocirrhosis, and hepatitis caused by viruses or drugs. As a result, hepatic disease complications such as portal hypertension may be prevented or treated, but the disease is not limited thereto. More specifically, the pharmaceutical composition according to the present invention is also effective for the treatment or prevention of hepatic diseases selected among liver transplantation, alcoholic or nonalcoholic fatty liver (see Korean Patent No. 10-2006247), hepatic fibrosis, hepatocirrhosis, hepatitis caused by viruses or drugs, and effective for alcoholic acute and chronic hepatic diseases. Further, the composition according to the present invention is effective for the treatment or prevention of fatty liver derived from fatty acids, or acute and chronic hepatic diseases derived from fatty liver.

According to Korean Patent No. 10-1852304, the compound of Chemical Formula 1 may enhance differentiation efficiency and maturity of stem cell-derived cardiomyocytes by comprising the step of culturing stem cells.

In addition, according to WO2016-072692, the compound of Chemical Formula 1 can also be used for the prevention and treatment of mucositis.

The present invention also provides a method for preventing or treating nontuberculous mycobacterium infectious diseases, the method comprising: administering the pharmaceutical composition according to the present invention to a subject in need thereof.

As used herein, the nontuberculous mycobacteria (NTM) refers to all mycobacteria except for the Mycobacterium complex and *M. leprae,* and may be, for example, at least one selected from the group consisting of *Mycobacterium avium, Mycobacterium intracellulare, Mycobacterium kansasii, Mycobacterium fortuitum* and *Mycobacterium abscessus,* preferably *Mycobacterium abscessus,* but is not limited thereto.

Specifically, in the present invention, nontuberculous mycobacterium infectious diseases may comprise one or more selected from the group consisting of lung diseases, lymphadenitis, skin/soft tissue/bone infections and disseminated diseases, but is not limited as long as it is a symptom that appears due to nontuberculous mycobacterium infection.

In the related art, it is known that two injection drugs need to be used together with one of amikacin, imipenem, and cefoxitin together with clarithromycin against lung diseases caused by a *Mycobacterium abscessus* strain.

Therefore, the compound represented by Chemical Formula 1 of the present invention or a pharmaceutically acceptable salt thereof may be used in combination with clarithromycin; and one or more drugs selected from the group consisting of amikacin, imipenem and cefoxitin.

As used herein, "treatment" refers to stopping or delaying the progress of the disease when used for a subject who shows the symptoms of the onset of the disease, and "prevention" refers to stopping or delaying the symptoms of the onset of the disease when used for a subject that does not show, but is at risk of, the symptoms of the onset of the disease.

In the present invention, the pharmaceutical composition may contain a pharmaceutically acceptable carrier together with the compound of the present invention, if necessary.

The compound of Chemical Formula 1 according to the present invention may be administered in various oral and parenteral dosage forms, and during formulation, the compound of Chemical Formula 1 according to the present invention is prepared using a diluent or an excipient, such as a filler, an extender, a binder, a wetting agent, a disintegrant, and a surfactant, which are commonly used.

A solid formulation for oral administration comprises a tablet, a pill, a powder, a granule, a capsule, a troche, and the like, and the solid formulation is prepared by mixing at least one excipient, for example, starch, calcium carbonate, sucrose or lactose, gelatin, and the like with one or more compounds of the present invention. Further, in addition to a simple excipient, lubricants such as magnesium stearate and talc are also used. A liquid preparation for oral administration corresponds to a suspension, a liquid for internal use, an emulsion, a syrup, and the like, and the liquid preparation may comprise various excipients, for example, a wetting agent, a sweetener, an aroma, a preservative, and the like, in addition to water and liquid paraffin, which are commonly used simple diluents.

A preparation for parenteral administration comprises an aqueous sterile solution, a non-aqueous solvent, a suspension solvent, an emulsion, a freeze-dried preparation, a suppository, or the like. As a non-aqueous solvent and a suspension solvent, it is possible to use propylene glycol, polyethylene glycol, a vegetable oil such as olive oil, an injectable ester such as ethyl oleate, and the like. As a base of the suppository, it is possible to use Witepsol, Macrogol, Tween 61, cacao butter, laurin fat, glycerol, gelatin, and the like.

In addition, the effective dosage of the compound of Chemical Formula 1 of the present invention for the human body may vary depending on the patient's age, body weight, sex, administration form, health condition, and severity of disease, and is generally about 0.001 to 100 mg/kg/day, preferably 0.01 to 35 mg/kg/day. Based on an adult patient weighing 70 kg, the dosage is generally 0.07 to 7000 mg/day, preferably 0.7 to 2500 mg/day, and the compound of the present invention may be administered once or in several divided doses a day at regular time intervals according to the judgment of a doctor or pharmacist.

In the present invention, when the pharmaceutical composition is formulated as a drug, reference may be made to the content disclosed in Remington's Pharmaceutical Sciences, Mack Publishing Company, Easton PA, which is incorporated herein by reference.

The term "subject" of the present invention includes animals such as horses, sheep, pigs, goats, camels, antelopes, and dogs, or humans, which have a nontuberculous mycobacterium infectious disease whose symptoms can be alleviated by administration of the pharmaceutical composition according to the present invention. By administering the pharmaceutical composition for use in the prevention or treatment according to the present invention to a subject, nontuberculous mycobacterium infectious diseases may be effectively prevented or treated.

As used herein, the "administration" refers to introduction of a predetermined material to a human or animal by any appropriate method, and for the route of administration of the therapeutic composition according to the present invention, the preventive or therapeutic composition according to the present invention may be orally or parenterally administered via any general route, which may reach a target tissue.

Numerical values set forth herein should be construed to comprise a range of equivalents, unless otherwise specified.

### [Advantageous Effects]

The compound represented by Chemical Formula 1 according to the present invention or a pharmaceutically acceptable salt thereof can be used for use in the prevention or treatment of nontuberculous mycobacterium (NTM) infectious diseases.

### [Description of Drawings]

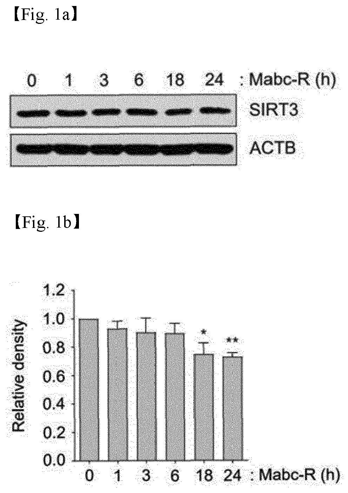

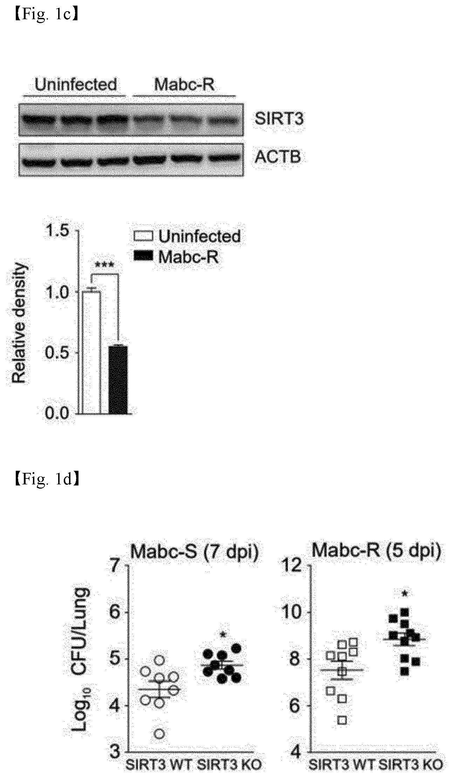

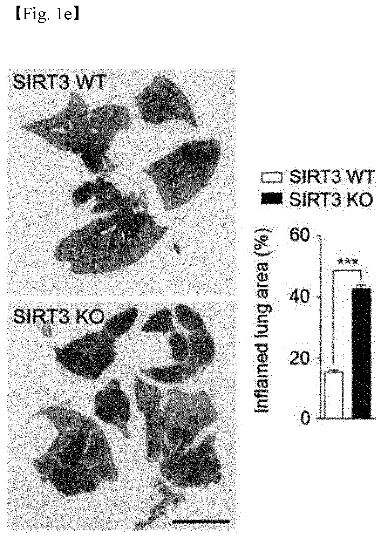

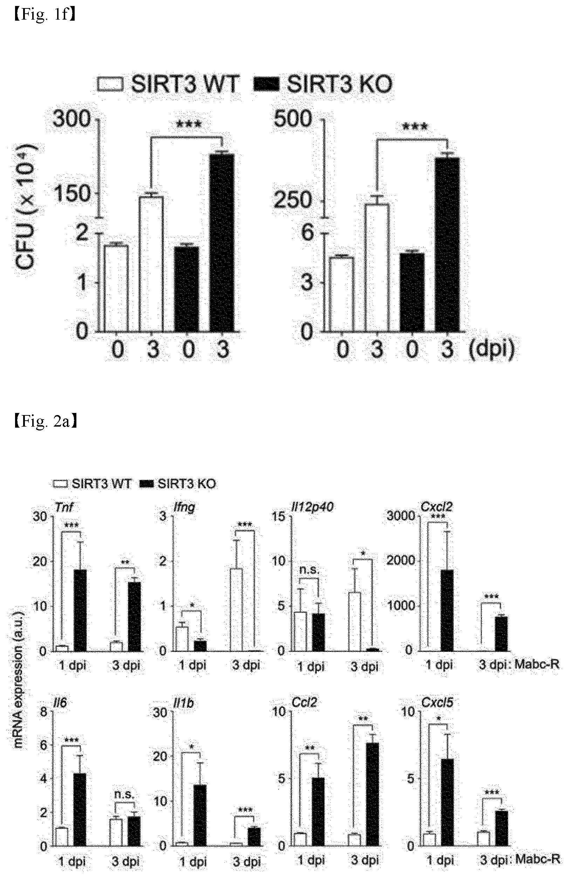

FIG. 1 shows that SIRT3 is essential for host defense against mycobacterial infection *in vivo* and *in vitro* (A and B). WT BMDMs were infected with Mabc-R (MOI=3) at the indicated times and actin protein levels were evaluated by immunoblotting as an internal control. B shows the results of quantifying A. In C, Western blot analysis for the lung tissues from SIRT3 WT and KO mice left uninfected or infected intranasally with Mabc-R (1 × 10⁷ CFU) for 3 days. D shows the results in which SIRT3 WT and KO mice were infected intranasally with various CFUs of Mabc-R (1 × 10⁷ CFU) or Mabc-S (1 × 10⁷ CFU) and monitored at 5 or 7 days post-infection (dpi). Data is shown as log pulmonary CFU. E shows lung histopathology by H&E staining of SIRT3 WT and KO mice infected with Mabc-R for 5 days. (right, quantification of results on left, scale bar, 5mm). F shows the intracellular survival results of Mabc-S assessed by a CFU assay. SIRT3 WT and KO BMDMs were infected with Mabc-S (MOI = 1 for the left; MOI = 3 for the right) for 4 hours, and then lysed to determine intracellular bacterial loads at 0 and 3 dpi. *P < 0.05, **P < 0.01, ***P < 0.001. Non-parametric test (FIGS. 1B and 1D); Student's t-test (C bottom and E right); One-way ANOVA (F). Data represents three independent experiments (A and C top, and E left), and values represent means (± SEM) from three or four independent experiments performed in triplicate (B and C bottom, E right, and F).

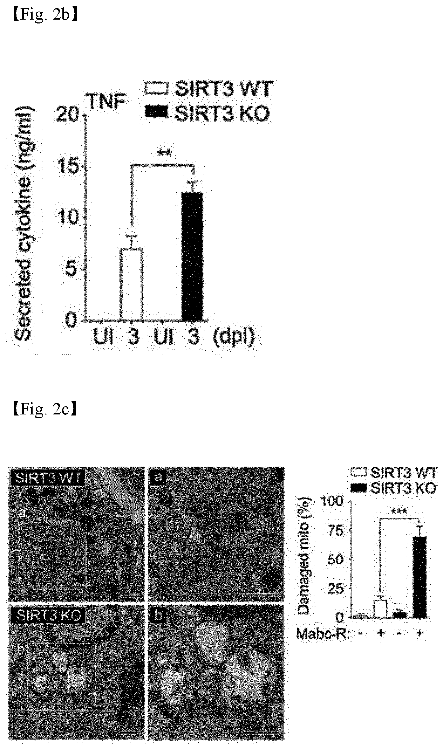

FIG. 2 shows that SIRT3 is required to control pathological inflammation and mitochondrial damage during Mabc-R infection. (A and B). SIRT3 WT and KO mice (n = 8 each group) were infected intranasally with Mabc-R (1 × 10⁷ CFU) and monitored at 1 and 3 dpi. A shows the results of subjecting lung tissues to quantitative real-time PCR analysis for the measurement of mRNA expression of various cytokines/chemokines. B shows the results of applying the supernatants from lung lysates to ELISA analysis of TNF (at 3 dpi) (UI: uninfected). C shows the results in which SIRT3 WT and KO mice (n = 3 each group) were infected intranasally with Mabc-R (1 × 10⁷ CFU) and monitored at 5 dpi. The lung tissues were harvested and then subjected to TEM analysis (left). Mitochondria with complete cristae are shown as follows. Swollen mitochondria with vacuolation in the cristae are shown in B. Right shows the quantitative analysis of at least 8 EM images in the lung tissues from SIRT3 WT and KO mice infected intranasally with Mabc-R (1 × 10⁷ CFU; n = 3 each group). The ratio of damaged mitochondria among total mitochondria was calculated quantitatively. (scale bars, 500 nm. *P < 0.05, **P < 0.01, ^{∗∗∗}P < 0.001, n.s., not significant compared with SIRT3 WT conditions; Non-parametric test; data represents three independent experiments (C left), and values represent means (± SEM) from three or four independent experiments performed in triplicate)

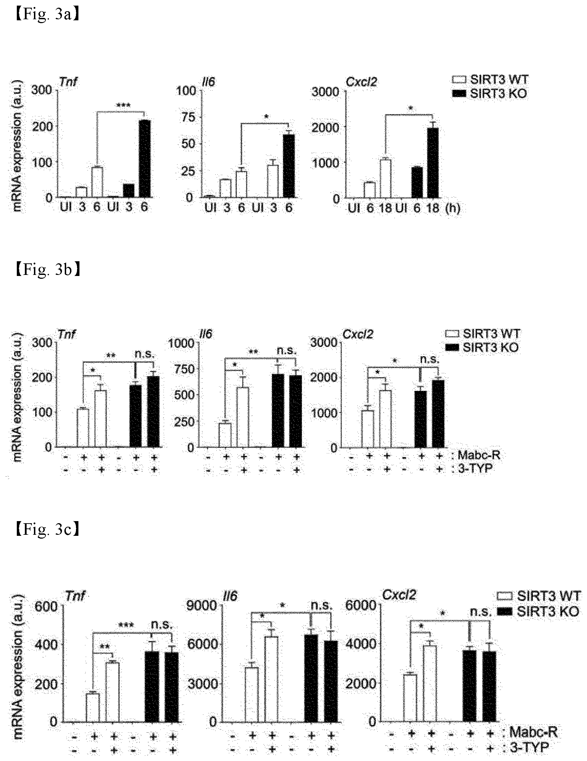

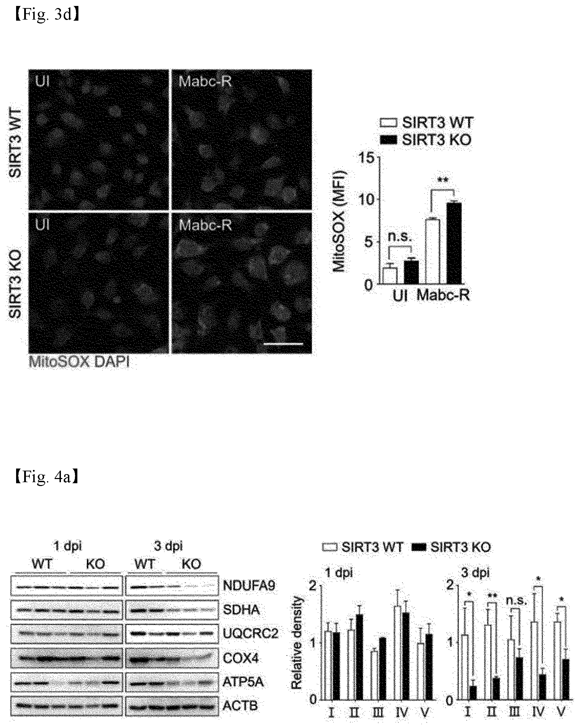

FIG. 3 shows that SIRT3 is essential for the amelioration of proinflammatory cytokine expression and controlling mitochondrial ROS production in BMDMs during Mabc-R infection. A shows the results that BMDMs of SIRT3 WT and KO mice were infected with Mabc-R (MOI = 3) and incubated for 3, 6, or 18 hours (B and C). BMDMs of B and PMs of C were prepared from SIRT3 WT and KO mice, and were infected with Mabc-R (MOI = 3) in the presence or absence of 3-TYP (50 µM) for 3 hours. Quantitative real-time PCR analysis for *Tnf, Il6,* and *Cxcl2* was performed. D shows that SIRT3 WT and KO BMDM were infected with Mabc-R (MOI = 3) for 2 hours and subjected to MitoSOX Red staining (representative images are shown in the left panel and quantitative analysis is shown in the right panel). (Scale bar, 50 µm. *P < 0.05, **P < 0.01, ***P < 0.001. UI, uninfected. One-way ANOVA (A to D). Data is representative of three independent experiments (D left), and values represent means (± SEM) from three or four independent experiments performed in triplicate (A to C, D right))

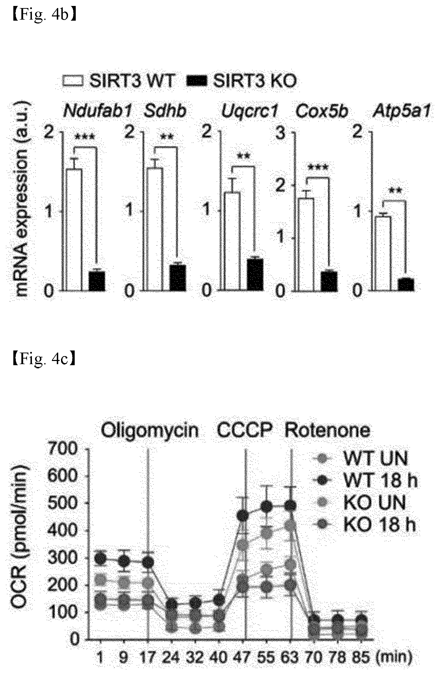

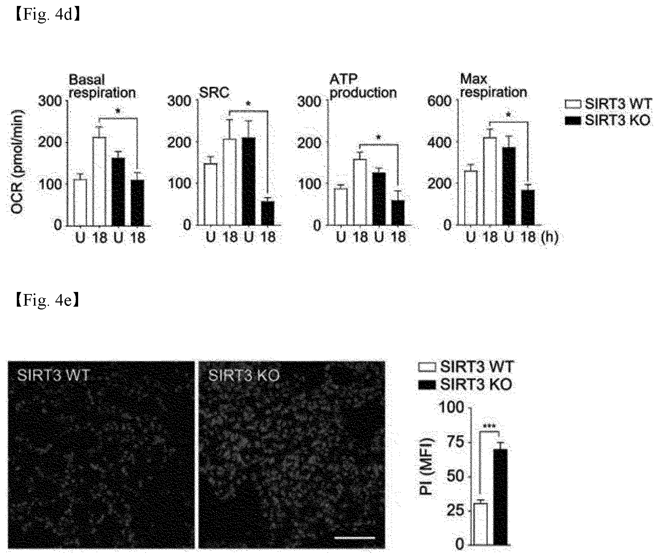

FIG. 4 shows that SIRT3 is required for mitochondrial OXPHOS function and attenuates cell death during Mabc-R infection (A and B). SIRT3 WT and KO mice (n = 8 each group) were infected intranasally with Mabc-R (1 × 10⁷ CFU) and monitored at 1 and 3 dpi. Lung tissues were collected and subjected to Western blot analysis for measurement of OXPHOS protein expression (left, representative images; right, quantitative analysis) and qRT-PCR analysis (A and B). C shows the oxygen consumption rate (OCR) analysis results of SIRT3 WT and KO BMDMs untreated (WT UN or KO UN) or treated (WT 18h or KO 18h) with Mabc-R for 18 hours. D shows quantitative analysis of basal respiration, spare respiratory capacity (SRC), ATP production, and maximal respiration analysis from C, and E shows PI staining after infection (left, representative images; right, quantitative analysis). (scale bar, 300 µm. *P < 0.05, **P < 0.01, ^{∗∗∗}P < 0.001. n.s., not significant. Paired t-test (right); non-parametric test (B and E right); One-way ANOVA (D). Data represents three independent experiments (A and C left, and E left), and values represent means (± SEM) from three or four independent experiments performed in triplicate (A right, B, D, and E right).

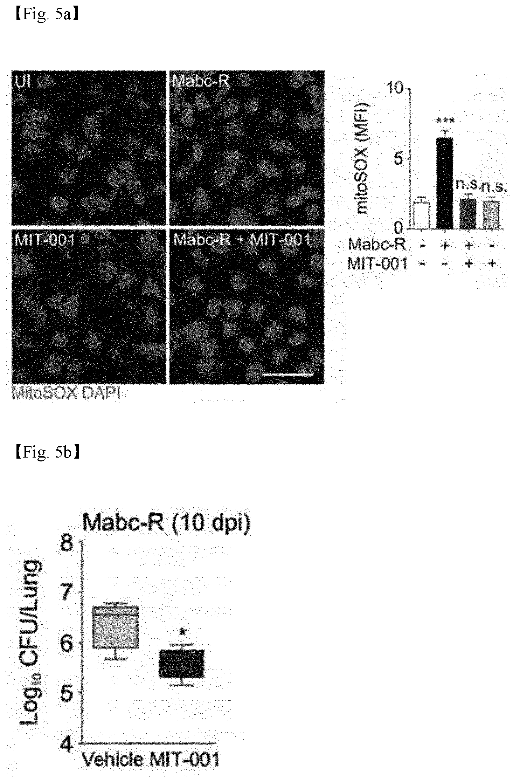

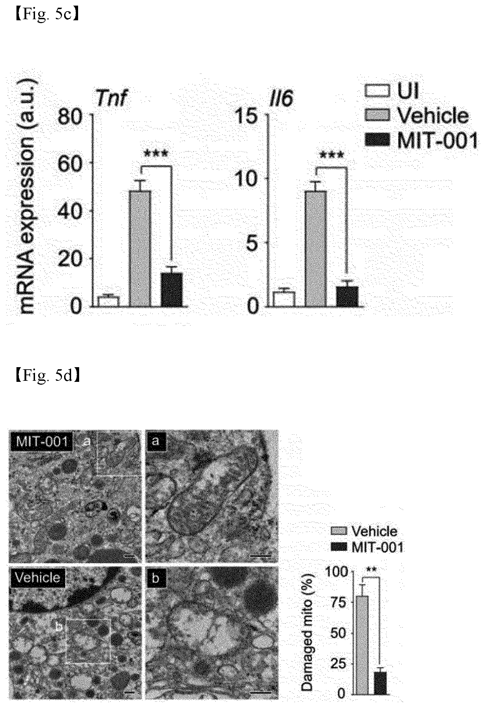

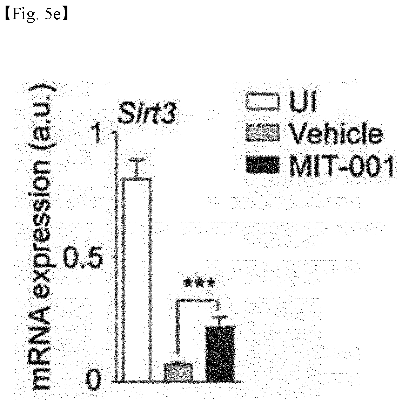

FIG. 5 shows that administration of the composition represented by Chemical Formula 1 to mice led to a protective effect against Mabc-R infection. A shows the results of infecting WT BMDMs with Mabc-R (MOI = 5) for 2 hours in the presence or absence of the compound represented by Chemical Formula 1 (20 µM) and subjecting the same to MitoSOX Red staining (left, representative images; right, quantitative analysis). Scale bar, 50µm. (B to E) WT mice (n = 5 each group) were left uninfected or infected intranasally with Mabc-R (1 × 10⁷ CFU), prior to treatment with or without the compound represented by Chemical Formula 1 (30 mg/kg) and monitored at 10 days post-infection. Lung tissues were subjected to pulmonary CFU assay in B, qRT-PCR analysis for cytokines/chemokines in C, and TEM analysis in D. Mitochondria with complete cristae are shown in a of D, and swollen mitochondria with vacuolation in the cristae are shown in b of D. Right shows the quantitative analysis of at least 8 EM images in the lung tissues from each group of mice infected intranasally with Mabc-R (1 × 10⁷ CFU; n = 3 each group). The ratio of damaged mitochondria among total mitochondria was calculated quantitatively (scale bars, 200 nm). E shows qRT-PCR analysis results for Sirt3 mRNA expression. (*P < 0.05, **P < 0.01, ^{∗∗∗}P < 0.001, n.s., not significant. One-way ANOVA (right) or non-parametric test (B, C, D right and E)). (Data represents three independent experiments (A left and D), and values represent means (± SEM) from three or four independent experiments performed in triplicate (A right, B, C, and E))

### [Modes of the Invention]

The benefits and features of the present invention, and the methods of achieving the benefits and features will become apparent with reference to experimental examples and preparation examples to be described below in detail. However, the present invention is not limited to the experimental examples and the preparation examples to be disclosed below and may be implemented in various other forms, and the present invention is provided for rendering the disclosure of the present invention complete and for fully informing the scope of the present invention to a person with ordinary skill in the art to which the present invention pertains.

In the following Examples, the compound represented by Chemical Formula 1 according to the present specification is described as MIT-001.

### [Examples]

### Materials and experimental methods

### 1. Mycobacterial strains and inoculum preparation for infection

In the present invention, the smooth-morphotype Mabc ATCC19977 WT (Mabc-S) strain and the isogenic rough type (Mabc-R) strain were used. This genetically identical rough type of Mabc ATCC19977 strain was obtained through continuous anaerobic passage of the WT strain and has previously been used to study Mabc-R virulence factors. The bacteria were incubated at 37°C using an orbital shaker (Middlebrook 7H9) in Middlebrook 7H9 medium containing 10% oleic albumin dextrose catalase (BD, Franklin Lakes, NJ) until the mid-log phase (OD₆₀₀ₙₘ = 0.6).

After cultivation, bacterial culture broths were harvested by centrifugation, and a bacterial pellet was washed three times with a PBS buffer solution to completely remove glycerol and BSA. Since these bacteria, particularly Mabc-R, grow into cord-formatted clumps, they were separated into single cells at 3000 rpm for 2 minutes using a tissue homogenizer consisting of a Teflon rod and a glass tube (Wheaton, Millville, NJ, USA). After the separation process, the bacterial single-cell suspensions were aliquoted and stored at -70°C until just before use. Prior to use, the frozen bacterial stocks were thawed in ice and immersed in an ultrasonic bath (As-one, Osaka, Japan) to prevent re-clumping and were used for the infection procedure.

Mabc CIP 104536^{T} R and S strains were kindly provided by Dr. Laurent Kremer (Universite de Montpellier, Montpellier, France). Mabc CIP 104536^{T} R and S morphotypes carrying a pMV262-mWasabi plasmid that enables the expression of mWasabi were used for the evaluation of bacterial dissemination of ZF. Mid-log phase Mabc strains were harvested and declumped before preparing a frozen stock using a 26-gauge needle and sonication was performed at 40 kHz for 30 seconds three times (Branson CPX3800, Danbury, CT, USA). Prior to injection, the numbers of viable bacteria were enumerated by plating serially diluted stocks on 7H10 agar. For ZF infection, the inoculum was diluted with phosphate-buffered saline-Tween 20 (PBST) 0.05% Tween 80 and resuspended in phenol red 0.085% to obtain 130 CFU/mL.

### 2. Mycobacterial infection of mice

Mycobacterial infection of SIRT3 WT and KO mice was performed as previously described. Groups of male 6 to 8-week-old mice were infected intranasally with Mabc-R (1 × 10⁶ or 10⁷ CFU/mouse) or Mabc-S (1 × 10⁷ CFU/mouse) for 5 to 7 days. At 24 hours post-infection, the numbers of bacteria in the lungs of at least three mice were determined to confirm the Mabc-R and Mabc-S inoculum, and represent the average inoculum of mice in each group.

### 3. Maintenance of mice and isolation of macrophages

SIRT3 WT and KO mice were kindly provided by Dr. Hyun Seok Kim (Ewha Womans University, Seoul, Korea) and maintained under specific-pathogen-free conditions at the Chungnam National University School of Medicine. The mice used in all experiments were 6 to 8 weeks old and sex-matched. All mice were bred and housed for experiments in accordance with the guidelines of the Chungnam National University School of Medicine. Mice experimental protocols were approved by the Institutional Animal Care and Use Committee of Chungnam National University (CNUA-18-0117).

Primary mouse bone marrow-derived macrophages (BMDMs) were isolated and cultured as previously described. BMDMs were isolated and differentiated after culture for 4 to 5 days in medium containing 25 ng/mL macrophage colony-stimulating factor (R&D Systems, Minneapolis, MIN, USA). Peritoneal macrophages (PMs) were isolated 2 to 3 days after injection with 1 mL of PBS supplemented with 3% thioglycollate (Sigma-Aldrich, St. Louis, MO, USA). Prior to isolation, PMs in the mouse abdominal cavity were collected with pre-chilled PBS supplemented with 10% FBS. BMDMs and PMs were cultured in a medium consisting of DMEM supplemented with 10% FBS, and penicillin-streptomycin-amphotericin B in a 5% CO₂ atmosphere at 37°C.

### 4. Antibodies and chemicals

MIT-001 (C25H32N4O4S2; Patent No. KR2008-0080519) was provided by MitoImmune Therapeutics, Inc. (Seoul, Korea). Antibodies against SIRT3 (5490S) and ACTB (sc-47778) were purchased from Cell Signaling Technology (Danvers, MA, USA) and Santa Cruz Biotechnology (Dallas, TX, USA), respectively. Antibodies against NDUFA9 (ab14713) and UQCRC2 (ab14745) were purchased from Abeam (Cambridge, UK), antibodies against SDHA (5839) and COX4 (4844) were purchased from Cell Signaling Technology (Danvers, MA, USA), and antibodies against ATP5A (459240) were purchased from Thermo Fisher Scientific. A propidium iodide (PI) solution (P3566) and MitoSOX^{™} Red (M36008) were purchased from Invitrogen (Carlsbad, CA, USA). 3-TYP (S8628) was purchased from Selleckchem. Resveratrol was synthesized from 1-ethynyl-3,5-dimethoxybenzene, as described in Reference [20].

### 5. Determination of CFUs from Mabc-infected lungs and macrophages

For *in vivo* CFU assays, lungs of the infected mice were harvested on day 5 or 7, depending on the experimental design. For the measurement of the bacterial burden, the lungs were homogenized in PBST and serial dilutions of the homogenates were plated on duplicate plates of Middlebrook 7H10 agar.

For the quantification of intracellular bacteria from macrophages, CFU assays were performed as previously described in Reference [21]. Briefly, Mabc-infected BMDMs were washed with PBS, and a fresh medium containing 50 µg/mL gentamicin (Sigma-Aldrich, St. Louis, MO, USA) was added and cell lysis was performed with 0.3% saponin (Sigma-Aldrich) to release intracellular bacteria. Then, infected lysates were vigorously resuspended, transferred to screw-capped tubes, and sonicated in a preheated 37°C water bath sonicator (Elma, Singen, Germany) for 5 minutes. Aliquots of the sonicated lysates were diluted 5-fold in 7H9 medium and homogenates were plated on duplicate plates of Middlebrook 7H10 agar. Bacterial colonies were counted after 3 to 5 days of incubation at 37°C.

### 6. Immunohistochemistry (IHC) and PI staining for Mabc-infected lung tissues

Lungs were harvested from mice infected with Mabc for 10 days. The lungs were fixed in 10% formalin and embedded in paraffin wax. For histopathology, lung paraffin sections (4 µm) were cut and stained with hematoxylin and eosin (H&E) as described in Reference [21]. For analysis of the extent of tissue necrosis, PI staining was performed. For analysis of the extent of tissue necrosis, lung paraffin sections (4 µm) were cut and immunostained with a propidium iodide solution (P3566; Invitrogen, Carlsbad, CA, USA). After initiation, fluorescence images were obtained using a confocal laser-scanning microscope (LSM 710; Zeiss, CLSM, Jena, Germany), with constant excitation, emission, pinhole, and exposure-time parameters. H&E staining was scanned using an Aperio digital pathology slide scanner (Leica) and imaged using an Aperio ScanScope^{®} CS System. To quantify the inflamed area and necrosis, the MFI of the red threshold was determined using FIJI software.

### 7. RNA extraction and quantitative real-time PCR (qPCR) analysis

RNA extraction and qPCR were performed as described in Reference [21]. Briefly, total RNA from BMDMs or lung tissues was isolated using the TRIzol reagent (Thermo Fisher Scientific). cDNA synthesis was performed using Superscript II reverse transcriptase (Invitrogen, 18064). qPCR was performed using cDNA, primers, and SYBR Green PCR Kits (Qiagen, 204074) using a Real-time PCR Cycler Rotor-Gene Q 2plex system (Qiagen GmbH, 9001620, Hilden, Germany). The samples were amplified for 50 cycles as follows: 95°C for 5 seconds and 60°C for 10 seconds. To analyze qPCR data, relative quantification was performed using the 2^{ΔΔ}Ct method using *Gapdh* as an internal control gene. Data was expressed as a relative fold change. The primer sequences are shown in Table 1.

**[Table 1]**

| Gene | Primer | Sequence |
|---|---|---|
| *Tnf* | Forward | 5'-ACGGCATGGATCTCAAAGAC-3' |
| | Reverse | 5'-AGATAGCAAATCGGCTGACG-3' |
| *Il6* | Forward | 5'-TACCACTTCACAAGTCGGAGGC-3' |
| | Reverse | 5'-CTGCAAGTGCATCATCGTTGTTC-3' |
| *Il12p40* | Forward | 5'-TTGAACTGGCGTTGGAAGCACG-3' |
| | Reverse | 5'-CCACCTGTGAGTTCTTCAAAGGC-3' |
| *Il1b* | Forward | 5'-TACGGACCGCAAAAGATGA-3' |
| | Reverse | 5'-TGCTGCTGCGAGATTTGAAG-3' |
| *Ifng* | Forward | 5'-CGGCACAGTCATTGAAAGCC-3' |
| | Reverse | 5'-TGCATCCTTTTTCGCCTTGC-3' |
| *Ccl2* | Forward | 5'-TGACCCCAAGAAGGAATGGG-3' |
| | Reverse | 5'-ACCTTAGGGCAGATGCAGTT-3' |
| *Cxcl2* | Forward | 5'-CCCTGCCAAGGGTTGACTTC-3' |
| | Reverse | 5'-GCAAACTTTTTGACCGCCCT-3' |
| *Cxcl5* | Forward | 5'-CCGCTGGCATTTCTGTTGCTGT-3' |
| | Reverse | 5'-CAGGGATCACCTCCAAATTAGCG-3' |
| *Sirt3* | Forward | 5'-GCTACATGCACGGTCTGTCGAA-3' |
| | Reverse | 5'-CAATGTCGGGTTTCACAACGCC-3' |
| *Ndufab1* | Forward | 5'-GGACCGAGTTCTGTATGTCTTG-3' |
| | Reverse | 5'-AAACCCAAATTCGTCTTCCATG-3' |
| *Sdhb* | Forward | 5'-ACCCCTTCTCTGTCTACCG-3' |
| | Reverse | 5'-AATGCTCGCTTCTCCTTGTAG-3' |
| *Uqcrc1* | Forward | 5'-ATCAAGGCACTGTCCAAGG-3' |
| | Reverse | 5'-TCATTTTCCTGCATCTCCCG-3' |
| *Cox5b* | Forward | 5'-ACCCTAATCTAGTCCCGTCC-3' |
| | Reverse | 5'-CAGCCAAAACCAGATGACAG-3' |
| *Atp5a1* | Forward | 5'-CATTGGTGATGGTATTGCGC-3' |
| | Reverse | 5'-TCCCAAACACGACAACTCC-3' |
| *Gapdh* | Forward | 5'-AAGATGGTGATGGGCTTCCCG-3' |
| | Reverse | 5'-TGGCAAAGTGGAGATTGTTGCC-3' |

### 8. Enzyme-linked immunosorbent assay (ELISA)

The concentration of TNF in lung tissues was measured using a commercially available ELISA kit (BD Biosciences, 558534, San Jose, CA, USA). Experiments were performed according to the protocol provided by the manufacturer.

### 9. Western blot analysis

Tissue homogenates were lysed in radioimmunoprecipitation assay (RIPA) buffer (50 mM Tris-HCl, pH 7.5, 150 mM NaCl, 1% NP-40, 0.5% deoxycholic acid (DOC), 0.1% SDS, and 1 mM PMSF) (ELPIS Bio, Lexington, MA, USA) supplemented with protease and phosphatase inhibitor cocktail (Roche, Basel, Switzerland). Equal amounts of protein were mixed with 5X SDS sample buffer (ELPIS) and heated for 5 minutes. Proteins were then separated by sodium dodecyl sulfate-polyacrylamide gel electrophoresis (SDS-PAGE) and transferred to polyvinylidene difluoride (PVDF) membranes (Millipore, Burlington, MA, USA). The membranes were blocked in 5% skim milk in Tris-buffered saline-Tween 20 (TBS-T) at room temperature for 1 hour, and were incubated at 4°C overnight with the following specific primary antibodies: anti-SIRT3 (5490, Cell Signaling Technology), anti-ACTB (sc-47778, Santa Cruz Biotechnology), anti-NDUFA9 (ab14713, Abeam, Cambridge, UK), anti-SDHA (5839, Cell Signaling Technology), anti-UQCRC2 (ab14745, Abeam), anti-COX4 (4844, Cell Signaling Technology), anti-ATP5A (459240, Thermo Fisher Scientific). After washing with TBS-T, the membranes were incubated with a horseradish peroxidase-conjugated secondary antibody (Cell Signaling Technology) at room temperature for 1 hour. Blots were imaged using a chemiluminescence assay kit (Millipore) in a UVitec Alliance mini-chemiluminescence device (BioSPX, Abcoude, Netherlands). Band densities were quantified using ImageJ software, and data were normalized to β-actin loading control.

### 10. Measurement of mitochondrial ROS and immunofluorescence

Mitochondrial ROS were measured as described previously in Reference [16]. SIRT3 WT and KO BMDMs were incubated with 3 µM MitoSOX Red Mitochondrial Superoxide Indicator (Invitrogen, M36008). After 20 minutes, the cells were washed and measured using an immunofluorescence assay. Nuclei were stained by incubation with 4',6-diamidino-2-phenylindole (Sigma-Aldrich) at the same time. Immunofluorescence images were obtained using a confocal laser-scanning microscope (Zeiss). The mean fluorescence intensity (MFI) level of mitochondrial ROS was calculated for each sample. Each experiment was performed in triplicate, and at least 200 cells per well were counted.

### 11. Mitochondrial oxygen consumption rate (OCR) analysis

The XF24 biosensor cartridge was activated with 1 mL of XF24 calibrant solution (Seahorse Bioscience, Billerica, MA) per well at 37°C for 24 hours in a non-CO₂ incubation system. SIRT3 WT and KO BMDMs infected with Mabc for 18 hours were seeded at 2 × 10⁴ cells per well and incubated at 37°C for 24 hours. The cell plate was incubated at 37°C for 1 hour in a non-CO₂ incubation system after the addition of a 590 µl assay medium to each well. ATPase inhibitor oligomycin A (20 µg/mL, Sigma-Aldrich, MO, USA), uncoupler carbonyl cyanide 3-chlorophenylhydrazone (CCCP, 50 µM, Sigma-Aldrich, MO, USA), and mitochondrial complex I inhibitor rotenone (20 µM, Sigma-Aldrich, MO, USA) were sequentially added to each well after measurement of basal OCR. The oxygen consumption rate of the entire process was measured using a Seahorse Bioscience XF24 analyzer (Seahorse Bioscience, Billerica, MA).

### 12. Transmission electron microscopy (TEM) analysis

SIRT3 WT and KO mouse lung tissues were fixed with 2.5% glutaraldehyde in a 0.1 M cacodylate buffer (pH 7.2) containing 0.1% CaCl₂. After 3 hours, the cells were post-fixed with 1% OsO₄ in a 0.1 M sodium cacodylate buffer containing 0.1% CaCl₂ for 2 hours. The tissues were rinsed with cold distilled water and slowly dehydrated using an ethanol series and propylene oxide at 4°C. The tissues were embedded in Embed-812 and cured at 60°C for 30 hours. Ultrathin sections (70 nm) were cut with a diamond knife and an ULTRACUT UC7 ultramicrotome (Leica) and mounted on formvar-coated copper grids. Sections were stained with 4% uranyl acetate for 7 minutes and lead citrate for 7 minutes. TEM-stained sections were scanned using a Bio-High Voltage EM system (JEM-1400 Plus and JEM-1000 BEF; JEOL Ltd., Tokyo, Japan).

### 13. Statistical analysis

Statistical analysis was performed in Prism (GraphPad Software, v5.01, 2007). Data is presented as mean ± SEM (standard error of the mean). Data was analyzed using a two-tailed Student's t-test or non-parametric test. In the non-parametric test, two conditions were compared using a Mann-Whitney U-Test and three or more conditions were compared using one-way ANOVA with a Dunn's multiple comparison test where appropriate. Specific p values are described in detail in the figure legends. For the ZF survival study, Kaplan-Meier survival curves were generated and analyzed by Gehan-Breslow-Wilcoxon test.

### Results

### 1. SIRT3 is required for host defense against Mabc infection

Given the present inventors' previous findings of SIRT3 during Mtb infection (Reference [16]), the present inventors first investigated whether Mabc infection decreased the levels of SIRT3 in macrophages and *in vivo.* The present inventors found that Mabc-R infection significantly decreased the expression of SIRT3 in macrophages in a time-dependent manner (FIGS. 1A and 1B). When SIRT3 WT mice were intranasally infected with Mabc-R, SIRT3 protein levels were significantly reduced (~2 fold) at 1 day post-infection (dpi) in the lung tissues of the mice (FIG. 1C). Next, the present inventors investigated the role of SIRT3 in *in vivo* antimicrobial responses using SIRT3 WT and SIRT3 KO mice. SIRT3 WT and SIRT3 KO mice were intranasally infected with Mabc-R or Mabc-S. *In vivo* bacterial loads in the lungs were significantly higher in SIRT3 KO mice than in SIRT3 WT mice after infection with Mabc-R or Mabc-S (FIG. 1D). There was no significant difference in the inoculum dose of Mabc between the SIRT3 WT and KO mice.

Further, the SIRT3 KO mice had enhanced lung pathology at 5 dpi using Mabc-R (that is, granulomatous and inflammatory lesions in the lungs) compared to the SIRT3 WT mice (FIG. 1E). The intracellular survival assays revealed that the SIRT3 KO BMDMs had significantly higher intracellular Mabc-S (multiplicity of infection [MOI] = 1 and 3) than the SIRT3 WT BMDMs (FIG. 1F). Since Mabc-R infection significantly induced cell death at a MOI of 1 (2 dpi; data not shown), an intracellular survival assay using Mabc-R was not performed. Collectively, the above data indicates that SIRT3 contributes to antimicrobial responses against Mabc-R and Mabc-S infection.

### 2. SIRT3 is required for the amelioration of pathological inflammation and control of mitochondrial damage during Mabc-R infection

Since the Mabc-R variant is more virulent and active in the induction of inflammation than Mabc-S (Reference [8]), the present inventors next compared the lung inflammatory responses between SIRT3 WT and SIRT3 KO mice using the Mabc-R strain. To examine this, the present inventors collected lung tissues at 1 and 3 dpi, and performed qRT-PCR analysis for the mRNA levels of proinflammatory cytokines/chemokines. As illustrated in FIG. 2A, the mRNA expression levels of a variety of proinflammatory cytokines/chemokines (*Tnf, Il1b, 116, Cxcl2, Ccl2,* and *Cxcl5*) were significantly higher in lung tissues of SIRT3 KO mice than in those of SIRT3 WT mice at 1 and 3 dpi. The protein level of TNF was also upregulated in the lung tissues of the SIRT3 KO mice than in those of SIRT3 WT mice (FIG. 2B, 3dpi). However, both *Ifng* and *Il12p40* mRNA expression levels were remarkably lowered in the lungs of SIRT3 KO mice compared to those of SIRT3 WT mice (FIG. 2A).

It is well established that SIRT3 is essential in mitochondrial homeostasis and functions to protect various cells and tissues from stress-induced cell death (References [22 to 25]). In addition, the present inventors reported that SIRT3 is required for the maintenance of mitochondrial homeostasis during Mtb infection (Reference [16]). When the present inventors next performed ultrastructural analysis between SIRT3 WT and KO lungs after infection, the TEM data showed that SIRT3-deficient lungs had a marked accumulation of damaged mitochondria, as represented by swollen and disrupted cristae, when compared with SIRT3 WT mice at 5 dpi after Mabc-R infection (FIG. 2C). However, there was no significant difference in mitochondrial morphology between SIRT3 WT and KO lungs prior to infection (FIG. 2C right). The above data implies that SIRT3 deficiency results in a marked increase in mitochondrial damage and excessive inflammatory responses during Mabc-R infection.

### 3. SIRT3 deficiency increases inflammatory responses and mitochondrial oxidative stress in macrophages during Mabc-R infection

The present inventors next compared the mRNA expression of inflammatory cytokines in BMDMs of SIRT3 WT and SIRT3 KO mice after Mabc-R infection. Mabc-R-mediated mRNA generation of *Tnf, Il6,* and *Cxcl2* was significantly increased in BMDMs of SIRT3 KO mice compared to SIRT3 WT mice after infection in a time-dependent manner (FIG. 3A). The protein levels of TNF were also increased in SIRT3 KO BMDMs compared to SIRT3 WT BMDMs after Mabc-R infection (data not shown). Similarly, the present inventors performed qRT-PCR analysis of proinflammatory cytokines (*Tnf, Il6,* and *Cxcl2*) in both SIRT3 WT and SIRT3 KO BMDMs or PMs, which were pretreated with 3-TYP prior to Mabc-R infection. As shown in FIGS. 3B and 3C, the present inventors found that pretreatment of SIRT3 WT BMDMs or PMs with 3-TYP significantly increased the mRNA expression of *Tnf, 116,* and *Cxcl2* in response to Mabc-R. When compared with SIRT3 WT BMDMs or PMs, SIRT3 KO BMDMs or PMs showed significantly increased mRNA levels of *Tnf, Il6,* and *Cxcl2* after Mabc-R infection. However, 3-TYP pretreatment had no significant effect on mRNA expression of those proinflammatory cytokines in SIRT3 KO BMDMs or PMs, after infection with Mabc-R (FIGS. 3B and 3C). The above data strongly suggests that SIRT3 inhibition increases the expression levels of *Tnf, Il6,* and *Cxcl2* in macrophages after Mabc-R infection.

Previous studies demonstrated that mitochondrial ROS generation is regulated by SIRT3 in various cells (References [26 to 29]). Therefore, mitochondrial redox status was compared between SIRT3 WT and SIRT3 KO BMDMs using MitoSOX Red, a highly selective fluorescent probe for the detection of mitochondrial O₂⁻ (Reference [30]). The present inventors first examined whether Mabc-R generated more mitochondrial ROS in WT BMDMs than Mabc-S. The present inventors infected BMDMs with either Mabc-R or Mabc-S and found that Mabc-R led to more mitochondrial ROS production at 2 hours after infection than Mabc-S did. In addition, the present inventors found that mitochondrial O₂⁻ generation was significantly increased in SIRT3 KO BMDMs, when compared with SIRT3 WT BMDMs, after Mabc-R infection (FIG. 3D). The above data implies that SIRT3 deficiency expanded oxidative stress and upregulated proinflammatory cytokine expression in macrophages during Mabc-R infection.

### 4. SIRT3 is essential for the maintenance of oxidative phosphorylation function and blockade of exaggerated cell death during Mabc-R infection

The mitochondrial oxidative phosphorylation (OXPHOS) system is essential for energy production and cellular homeostasis (Reference [31]). The present inventors further determined the levels of major mitochondrial proteins in SIRT3 WT and KO lungs during Mabc-R infection. The present inventors found that the protein expression levels of OXPHOS were dramatically suppressed in the SIRT3 KO lungs, when compared with those of SIRT3 WT lungs, at 3 dpi after Mabc-R infection (I: NDUFA9, II: SDHA, III: UQCRC2, IV: COX4, V: ATP5A) (FIG. 4A). The present inventors then evaluated a differential expression profile of mitochondrial OXPHOS genes in the lungs of SIRT3 WT and KO mice infected with Mabc-R (FIG. 4B). Notably, the gene expression of mitochondrial OXPHOS was significantly decreased in the lungs of SIRT3 KO mice, when compared with those of SIRT3 WT mice, at 5 dpi after Mabc-R infection (FIG. 4B). The present inventors further analyzed bioenergetic characteristics in BMDMs of SIRT3 WT and SIRT3 KO mice by measuring OCR using the Seahorse XF24 analyzer (FIGS. 4C and 4D). In SIRT3 KO BMDMs, basal respiration, mitochondrial spare respiratory capacity, ATP production, and maximal respiration were significantly decreased, compared to those in SIRT3 WT BMDMs, after Mabc-R infection (FIGS. 4C and 4D). Combined with the data on mRNA and protein expression of mitochondrial respiratory chain complexes, the above data strongly suggests that SIRT3 is required for the maintenance of mitochondrial respiration during Mabc-R infection.

Given the findings that SIRT3 deficiency results in increased mitochondrial defects and ROS production, and reduced OXPHOS activity, the present inventors next examined whether cell death was upregulated in the lungs of SIRT3 KO mice compared to those of SIRT3 WT mice. The present inventors previously showed that Mabc-R variants induced greater cell death in RAW264.7 cells than the smooth strain did (Reference [18]). Therefore, the present inventors elucidated whether Mabc-R infection induced the activation of PI-positive cell death in infected lungs of mice. As illustrated in FIG. 4E, PI staining of lung tissues showed that cell death was remarkably increased in the lung tissues of SIRT3 KO mice compared to those of SIRT3 WT mice after Mabc-R infection. Furthermore, the above data implies that mitochondrial OXPHOS function was dramatically downregulated, but host cell death was remarkably upregulated in SIRT3 KO lungs during Mabc-R infection.

### 5. Blockade of excess mitochondrial ROS enhances the antimicrobial response and ameliorates pathological inflammation during Mabc-R infection

The mitochondrial ROS scavenger MIT-001 (previously, NecroX-7) removes mitochondrial ROS, calcium, and reactive nitrogen species (References [32 and 33]). As expected, treatment of BMDMs with MIT-001 remarkably suppressed the generation of mitochondrial ROS in response to Mabc-R infection (FIG. 5A). The present inventors next determined whether inhibition of mitochondrial ROS generation enhanced antimicrobial effects *in vivo.* Mice were intranasally infected with Mabc-R, treated with MIT-001 (at 1, 3, 5, 7, and 9 dpi), and sacrificed at 10 dpi for determination of *in vivo* CFUs. MIT-001 significantly inhibited the *in vivo* bacterial loads in the lungs of infected mice (FIG. 5B), implying that inhibition of excessive mitochondrial ROS is beneficial for controlling bacterial replication in response to Mabc infection.

We next examined the *in vivo* effects of MIT-001 on pathological inflammatory responses during Mabc-R infection. The mRNA expression of *Tnf* and *Il6* was dramatically increased in the mouse lungs after Mabc-R infection (FIG. 5C). The administration of MIT-001 remarkably ameliorated the expression of numerous inflammatory cytokines/chemokines, including *Tnf, Il1b, Il6, Cxcl2,* and *Cxcl5,* in the lung tissues of mice infected with Mabc-R (FIG. 5C). Further, TNF secretion was significantly downregulated in the lung tissues of Mabc-R-infected mice, by treatment with MIT-001.

To further analyze the mitochondrial damage *in vivo,* the present inventors performed TEM analysis of lung tissues of Mabc-R-infected mice regardless of MIT-001 treatment. After treatment with MIT-001, there were considerably fewer damaged mitochondria with disrupted cristae in the alveolar cells of lung tissues of Mabc-R-infected mice than in vehicle control mice (FIG. 5D). In addition, Sirt3 mRNA levels were remarkably suppressed in the mouse lungs after Mabc-R infection, and significantly recovered after MIT-001 treatment (FIG. 5E). Furthermore, the above data demonstrated that *in vivo* bacterial growth and pathological inflammation were significantly reduced by MIT-001 treatment, implying that controlling mitochondrial ROS is essential for promoting host defense during Mabc-R infection.

### [References]

[1] Davidson RM A closer look at the genomic variation of geographically diverse Mycobacterium abscessus clones that cause human infection and disease. Front Microbiol 2018;9:2988.
[2] Koh WJ, Stout JE, Yew WW Advances in the management of pulmonary disease due to Mycobacterium abscessus complex. Int J Tuberc Lung Dis 2014; 18: 1141-1148.
[3] Swenson C, Zerbe CS, Fennelly K Host variability in NTM disease: implications for research needs. Front Microbiol 2018;9:2901.
[4] Richards CJ, Olivier KN Nontuberculous mycobacteria in cystic fibrosis. Semin Respir Crit Care Med 2019;40:737-750.
[5] Prevots DR, Marras TK Epidemiology of human pulmonary infection with nontuberculous mycobacteria: a review. Clin Chest Med 2015;36:13-34.
[6] Sanchez-Chardi A, Olivares F, Byrd TF, et al. Demonstration of cord formation by rough Mycobacterium abscessus variants: implications for the clinical microbiology laboratory. J Clin Microbiol 2011;49:2293-2295.
[7] Ryan K, Byrd TF Mycobacterium abscessus: shapeshifter of the mycobacterial world. Front Microbiol 2018;9:2642.
[8] Ahmad M, Zeitlin IJ, Parratt JR, et al. Degradation of bradykinin, a cardioprotective substance, during a single passage through isolated rat-heart. Arch Pharm Res 2006;29:241-248.
[9] Chan ED, Iseman MD Underlying host risk factors for nontuberculous mycobacterial lung disease. Semin Respir Crit Care Med 2013;34:110-123.
[10] Storder J, Renard P, Arnould T Update on the role of Sirtuin 3 in cell differentiation: a major metabolic target that can be pharmacologically controlled. Biochem Pharmacol 2019;169:113621.
[11] Onyango P, Celic I, McCaffery JM, et al. SIRT3, a human SIR2 homologue, is an NAD-dependent deacetylase localized to mitochondria. Proc Natl Acad Sci USA 2002;99:13653-13658.
[12] Gomes P, Viana SD, Nunes S, et al. The yin and yang faces of the mitochondrial deacetylase sirtuin 3 in age-related disorders. Ageing Res Rev 2020;57:100983.
[13] Kurundkar D, Kurundkar AR, Bone NB, et al. SIRT3 diminishes inflammation and mitigates endotoxin-induced acute lung injury. JCI Insight 2019;4: e 120722.
[14] Paulin R, Dromparis P, Sutendra G, et al. Sirtuin 3 deficiency is associated with inhibited mitochondrial function and pulmonary arterial hypertension in rodents and humans. Cell Metab 2014;20:827-839.
[15] Ciarlo E, Heinonen T, Lugrin J, et al. Sirtuin 3 deficiency does not alter host defenses against bacterial and fungal infections. Sci Rep 2017;7:3853.
[16] Kim TS, Jin YB, Kim YS, et al. SIRT3 promotes antimycobacterial defenses by coordinating mitochondrial and autophagic functions. Autophagy. 2019;15:1356-1375.
[17] Heinonen T, Ciarlo E, Le Roy D, et al. Impact of the dual deletion of the mitochondrial Sirtuins SIRT3 and SIRT5 on anti-microbial host defenses. Front Immunol 2019;10:2341.
[18] Whang J, Back YW, Lee KI, et al. Mycobacterium abscessus glycopeptidolipids inhibit macrophage apoptosis and bacterial spreading by targeting mitochondrial cyclophilin D. Cell Death Dis 2017;8:e3012.
[19] Kim TH, Hanh BTB, Kim G, et al. Thiostrepton: A novel therapeutic drug candidate for Mycobacterium abscessus infection. Molecules. 2019;24:4511.
[20] Lara-Ochoa F, Sandoval-Minero LC, Espinosa-Perez G A new synthesis of resveratrol. Tetrahedron Lett 2015;56:5977-5979.
[21] Kim JK, Kim YS, Lee HM, et al. GABAergic signaling linked to autophagy enhances host protection against intracellular bacterial infections. Nat Commun 2018;9:4184.
[22] Ahn BH, Kim HS, Song S, et al. A role for the mitochondrial deacetylase Sirt3 in regulating energy homeostasis. Proc Natl Acad Sci USA 2008;105:14447-14452.
[23] Casey BJ, Somerville LH, Gotlib IH, et al. Behavioral and neural correlates of delay of gratification 40 years later: Proc. Natl. Acad. Sci. U.S.A. 2011, Vol 108 No. 36:14998-5003. Ann Neurosci. 2012;19: 27-28.
[24] Pillai VB, Bindu S, Sharp W, et al. Sirt3 protects mitochondrial DNA damage and blocks the development of doxorubicin-induced cardiomyopathy in mice. Am J Physiol Heart Circ Physiol 2016;310: H962-972.
[25] Sundaresan NR, Gupta M, Kim G, et al. Sirt3 blocks the cardiac hypertrophic response by augmenting Foxo3a-dependent antioxidant defense mechanisms in mice. J Clin Invest 2009;119:2758-2771.
[26] Oh JY, Choi GE, Lee HJ, et al. 17beta-Estradiol protects mesenchymal stem cells against high glucose-induced mitochondrial oxidants production via Nrf2/Sirt3/ MnSOD signaling. Free Radic Biol Med 2019;130:328-342.
[27] Feng J, Chen X, Liu R, et al. Melatonin protects against myocardial ischemia-reperfusion injury by elevating Sirtuin3 expression and manganese superoxide dismutase activity. Free Radic Res 2018;52:840-849.
[28] Torrens-Mas M, Hernandez-Lopez R, Oliver J, et al. Sirtuin 3 silencing improves oxaliplatin efficacy through acetylation of MnSOD in colon cancer. J Cell Physiol 2018;233:6067-6076.
[29] Zou X, Santa-Maria CA, O'Brien J, et al. Manganese superoxide dismutase acetylation and dysregulation, due to loss of SIRT3 activity, promote a luminal B-like breast carcinogenic-permissive phenotype. Antioxid Redox Signal 2016;25:326-336.
[30] Bulua AC, Simon A, Maddipati R, et al. Mitochondrial reactive oxygen species promote production of proinflammatory cytokines and are elevated in TNFR1-associated periodic syndrome (TRAPS). J Exp Med 2011;208:519-533.
[31] Castellanos E, Lanning NJ Phosphorylation of OXPHOS machinery subunits: functional implications in cell biology and disease. Yale J Biol Med 2019;92:523-531.
[32] Chung HK, Kim YK, Park JH, et al. The indole derivative NecroX-7 improves nonalcoholic steatohepatitis in ob/ob mice through suppression of mitochondrial ROS/RNS and inflammation. Liver Int 2015;35:1341-1353.
[33] Hwang IC, Kim JY, Kim JH, et al. Therapeutic potential of a novel necrosis inhibitor, 7-amino-indole, in myocardial ischemia-reperfusion injury. Hypertension 2018;71:1143-1155.
[34] Kim OY, Chung JY, Song J Effect of resveratrol on adipokines and myokines involved in fat browning: perspectives in healthy weight against obesity. Pharmacol Res 2019;148:104411.
[35] Hou CY, Tain YL, Yu HR, et al. The effects of resveratrol in the treatment of metabolic syndrome. Int J Mol Sci 2019;20:535.
[36] Kakoti BB, Hernandez-Ontiveros DG, Kataki MS, et al. Resveratrol and omega-3 fatty acid: its implications in cardiovascular diseases. Front Cardiovasc Med 2015;2:38.
[37] Marcus JM, Andrabi SA SIRT3 regulation under cellular stress: making sense of the ups and downs. Front Neurosci 2018;12:799.
[38] Caverly LJ, Caceres SM, Fratelli C, et al. Mycobacterium abscessus morphotype comparison in a murine model. PLoS One 2015;10:e0117657.
[39] Gutierrez AV, Viljoen A, Ghigo E, et al. Glycopeptidolipids, a double-edged sword of the Mycobacterium abscessus complex. Front Microbiol 2018;9:1145.
[40] Howard ST, Rhoades E, Recht J, et al. Spontaneous reversion of Mycobacterium abscessus from a smooth to a rough morphotype is associated with reduced expression of glycopeptidolipid and reacquisition of an invasive phenotype. Microbiology. 2006;152:1581-1590.
[41] Rhoades ER, Archambault AS, Greendyke R, et al. Mycobacterium abscessus glycopeptidolipids mask underlying cell wall phosphatidyl-myo-inositol mannosides blocking induction of human macrophage TNF-alpha by preventing interaction with TLR2. J Immunol 2009;183 :1997-2007.
[42] Davidson LB, Nessar R, Kempaiah P, et al. Mycobacterium abscessus glycopeptidolipid prevents respiratory epithelial TLR2 signaling as measured by HbetaD2 gene expression and IL-8 release. PLoS One 2011;6:e29148.
[43] Roca FJ, Ramakrishnan L TNF dually mediates resistance and susceptibility to mycobacteria via mitochondrial reactive oxygen species. Cell. 2013;153:521-534.
[44] To EE, Erlich JR, Liong F, et al. Mitochondrial reactive oxygen species contribute to pathological inflammation during influenza A virus infection in mice. Antioxid Redox Signal 2019;32:929-942.
[45] Belchamber KBR, Singh R, Batista CM, et al. Defective bacterial phagocytosis is associated with dysfunctional mitochondria in COPD macrophages. Eur Respir J 2019;54:1802244.
[46] Bewley MA, Preston JA, Mohasin M, et al. Impaired mitochondrial microbicidal responses in chronic obstructive pulmonary disease macrophages. Am J Respir Crit Care Med 2017;196:845-855.
[47] Bernut A, Nguyen-Chi M, Halloum I, et al. Mycobacterium abscessus-Induced granuloma formation is strictly dependent on TNF signaling and neutrophil trafficking. PLoS Pathog 2016;12:e1005986.
[48] Roca FJ, Whitworth LJ, Redmond S, et al. TNF Induces pathogenic programmed macrophage necrosis in tuberculosis through a mitochondrial-lysosomal-endoplasmic reticulum circuit. Cell. 2019;178:1344-1361 e1311.
[49] Bagul PK, Katare PB, Bugga P, et al. SIRT-3 modulation by resveratrol improves mitochondrial oxidative phosphorylation in diabetic heart through deacetylation of TFAM. Cells. 2018;7: pii: E235.
[50] Zumla A, Rao M, Parida SK, et al. Inflammation and tuberculosis: host-directed therapies. J Intern Med 2015;277:373-387.
[51] Mahon RN, Hafner R Immune cell regulatory pathways unexplored as host-directed therapeutic targets for Mycobacterium tuberculosis: an opportunity to apply precision medicine innovations to infectious diseases. Clin Infect Dis. 2015;61 Suppl 3:S200-216.
[52] Kiran D, Podell BK, Chambers M, et al. Host-directed therapy targeting the Mycobacterium tuberculosis granuloma: a review. Semin Immunopathol 2016; 38:167-183.
[53] Sun D, Hurdle JG, Lee R, et al. Evaluation of flavonoid and resveratrol chemical libraries reveals abyssinone II as a promising antibacterial lead. ChemMedChem 2012;7:1541-1545.
[54] Yang H, Hu J, Chen YJ, et al. Role of Sirt1 in innate immune mechanisms against Mycobacterium tuberculosis via the inhibition of TAK1 activation. Arch Biochem Biophys 2019;667:49-58.
[55] Kim SY, Yang CS, Lee HM, et al. ESRRA (estrogen-related receptor alpha) is a key coordinator of transcriptional and post-translational activation of autophagy to promote innate host defense. Autophagy. 2018;14:152-168.
[56] Pillai VB, Samant S, Sundaresan NR, et al. Honokiol blocks and reverses cardiac hypertrophy in mice by activating mitochondrial Sirt3. Nat Commun 2015;6:6656.

## Claims

1. A pharmaceutical composition for use in the prevention or treatment of nontuberculous mycobacterium infectious diseases, comprising, as an active ingredient, a compound represented by the following Chemical Formula 1 or a pharmaceutically acceptable salt thereof.

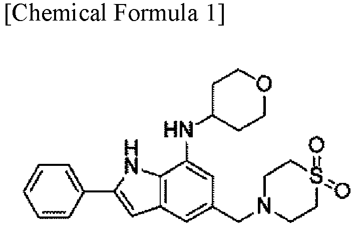

2. The pharmaceutical composition of claim 1, wherein the nontuberculous mycobacterium is *Mycobacterium abscessus.*

3. The pharmaceutical composition of claim 1, wherein the compound represented by Chemical Formula 1 or the pharmaceutically acceptable salt thereof is used in combination with clarithromycin; and one or more drugs selected from the group consisting of amikacin, imipenem and cefoxitin.

4. The pharmaceutical composition of claim 1, wherein the nontuberculous mycobacterium infectious disease comprises one or more selected from the group consisting of lung diseases, lymphadenitis, skin/soft tissue/bone infections and disseminated diseases.

5. The pharmaceutical composition of claim 1, wherein the pharmaceutical composition improves a SIRT3 mRNA level, which has decreased due to nontuberculous mycobacterium infection.

6. The pharmaceutical composition of claim 1, wherein the pharmaceutical composition is used in the prevention or treatment of the nontuberculous mycobacterium infectious disease for oral administration or parenteral administration.

7. The pharmaceutical composition of claim 1, wherein the pharmaceutical composition is formulated for injection.

8. A method for preventing or treating nontuberculous mycobacterium infectious diseases, the method comprising: administering the pharmaceutical composition of claim 1 to a subject in need thereof.
